# FASCICULE DE BREVET EUROPEEN

(11) **EP 3 090 042 B1**
(45) Date de publication et mention de la délivrance du brevet: **11.10.2017**
(21) Numéro de dépôt: 14831040.2
(22) Date de dépôt: 30.12.2014
(51) Int. Cl.: C12N 1/18, C12P 7/06, C12R 1/865

(54) **SOUCHES DE LEVURES POUR LA PRODUCTION D'ÉTHANOL DE PREMIÈRE GÉNÉRATION**
HEFESTÄMME ZUR HERSTELLUNG VON ETHANOL DER ERSTEN GENERATION
YEAST STRAINS FOR THE PRODUCTION OF FIRST GENERATION OF ETHANOL.

(30) Priorité: 30.12.2013 FR 1363672
(43) Date de publication de la demande: 09.11.2016
(73) Titulaire: Lesaffre et Compagnie, 75001 Paris (FR)
(72) Inventeur: BAVOUZET, Jean-Michel, F-59170 Croix (FR); QUIPOURT, Anne-Dominique, F-59700 Marc en Baroeul (FR); TBAIKHI, Annie, F-59118 Wambrechies (FR)
(74) Mandataire: Cabinet Plasseraud
(86) Numéro de dépôt international: PCT/FR2014/053575
(87) Numéro de publication internationale: WO 2015/101753

(56) Documents cités:
- WO-A1-98/31784
- WO-A1-2010/031916
- WO-A1-2011/100272
- BASSO LUIZ C ET AL: "Yeast selection for fuel ethanol production in Brazil", FEMS YEAST RESEARCH, vol. 8, no. 7, 22 août 2008 (2008-08-22), pages 1155-1163, XP002594694, ISSN: 1567-1356

## Description

### Domaine de l'Invention

La présente invention concerne des souches de levure productrices d'éthanol de première génération, des levures obtenues par culture de ces souches, et des procédés de production industrielle d'éthanol à partir desdites levures. Plus spécialement, la présente invention concerne trois souches spécifiques qui présentent une forte tolérance à l'éthanol, une plus faible production de glycérol que les souches les plus performantes utilisées aujourd'hui dans les procédés de production d'éthanol et un rendement élevé en éthanol. Les souches de l'invention disposent également d'une cinétique de production d'éthanol rapide.

### Contexte de l'Invention

La diminution des ressources en énergies non renouvelables et la préoccupation grandissante devant l'augmentation des émissions de gaz à effet de serre sont à l'origine de la nécessité de trouver des sources d'énergie alternatives aux carburants fossiles (pétrole, charbon, gaz). La biomasse végétale issue des forêts, des produits et/ou coproduits agricoles ou agro-alimentaires constitue une source de carbone considérable pour la production de molécules d'intérêt industriel. L'éthanol produit à partir des sucres fermentescibles contenus dans les végétaux est utilisé dans les véhicules dotés de moteurs à combustion. La production du bioéthanol a ainsi connu un développement rapide aux cours des dernières années en Amérique du Nord et en Europe. En 2008, plus de 56 milliards de litres d'éthanol ont été produits dans le monde à partir de biomasse végétale.

Le bioéthanol de première génération est produit par fermentation des hexoses (sucres à six carbones) contenus dans les biomasses riches en amidon (grains de maïs, orge, blé, manioc, tubercules de pomme de terre, etc.) ou en saccharose (canne à sucre, betterave sucrière, sorgho sucrier etc.), tandis que le bioéthanol dit de deuxième génération est produit par transformation de la cellulose et de l'hémicellulose contenues dans les résidus agricoles tels que les pailles de céréales, les cannes de maïs, les résidus forestiers, le bois, les cultures énergétiques telles que le panic érigé ou les taillis à courte ou très courte rotation (peuplier par exemple).

Les procédés d'obtention des milieux de fermentation riches en sucres des procédés de première génération sont relativement simples et bien maîtrisés. Dans le cas des plantes sucrières telles que la canne à sucre, le sorgho sucrier, la betterave sucrière, la plante est broyée ou coupée en morceaux et un jus sucré est obtenu directement ou après trempage dans de l'eau. La fermentation alcoolique peut avoir lieu à partir du jus brut obtenu, à partir du jus concentré ou de jus concentrés tels que les mélasses obtenus après extraction d'une fraction des sucres initialement présents. Dans le cas des fractions végétales riches en amidon telles que les grains de maïs, l'amidon doit être d'abord hydrolysé en glucose que la levure peut alors convertir en éthanol. Le procédé d'hydrolyse type consiste en une première phase au cours de laquelle les chaînes d'amidon sont converties en chaînes plus courtes par action d'une alpha-amylase suivie d'une étape de fermentation dite SSF (Saccharification et Fermentation Simultanées) au cours de laquelle les dextrines sont hydrolysées par ajout de glucosidases et au cours de laquelle le glucose est fermenté en éthanol par la levure. Certains procédés, dits procédés froids, réalisent une hydrolyse de l'amidon réduite voire nulle avant l'étape de fermentation. Dans le cas de l'éthanol de deuxième génération produit à partir de matière lignocellulosique, les procédés d'hydrolyse chimique et enzymatique sont beaucoup plus complexes et laborieux puisque la matière lignocellulosique est formée d'une matrice rigide difficile à déstructurer pour libérer la cellulose et l'hémicellulose de la lignine. L'hydrolyse des matériaux lignocellulosiques génère des hydrolysats contenant des hexoses et/ou des pentoses. Quelle que soit la biomasse utilisée ou le produit employé, le produit final est le même, seul le procédé de fabrication diffère.

En Europe, la betterave à sucre et les céréales (blé, orge, maïs) sont les principales ressources utilisées pour la production d'éthanol d'origine agricole. Les sucres (glucose, fructose ou saccharose) contenus dans les plantes sucrières (betterave à sucre, canne à sucre) et les plantes amylacées (céréales comme le blé ou le maïs) sont transformés en alcool par un procédé de fermentation industrielle utilisant des levures. L'alcool est ensuite distillé et déshydraté pour obtenir du bioéthanol. Les coproduits obtenus lors du processus de production (drêches et pulpes) sont destinés à l'alimentation animale.

Les levures utilisées par les producteurs d'éthanol de première génération sont principalement des levures spécialisées qui permettent d'optimiser la rentabilité du procédé de production. Ces levures sont entre autres: Ethanol Red^{®} (Fermentis^{®}), Thermosacc^{®} (Lallemand^{®}), Angel Super Alcohol^{®} (Angel^{®}) et Fali^{®} (AB Mauri^{®}). Les qualités attendues de ces levures sont leur capacité à produire rapidement de fortes concentrations en éthanol et d'épuiser les sucres des milieux de fermentation sur les plages de température et de pH représentatives des conditions industrielles. Ces qualités sont particulièrement recherchées dans les procédés utilisant les céréales, le maïs, en particulier, qui génèrent des hydrolysats à fortes concentrations en sucres. En effet, les producteurs ajustent la teneur en sucre de leur milieu de fermentation de façon à ce qu'elle soit la plus élevée possible tout en s'assurant que le sucre soit le plus rapidement et le plus complètement possible converti en éthanol. De la même façon que le producteur souhaite que la levure convertisse la totalité des sucres du milieu en éthanol, le producteur souhaite que le rendement global de conversion des sucres consommés en éthanol soit le plus élevé possible et en conséquence que le moins possible de coproduits tels que le glycérol soient générés au cours de la fermentation.

Disposer d'une levure présentant une meilleure tolérance à l'éthanol, disposer d'une levure produisant moins de glycérol tout en assurant une productivité volumique équivalent ou supérieure tant dans des conditions de fermentation habituelles que lors des fluctuations des paramètres de fermentation permettrait aux producteurs d'éthanol d'augmenter la rentabilité de leur installation en augmentant la production d'éthanol. Il existe donc toujours un besoin de disposer de nouvelles souches de levure améliorées pour la production d'éthanol de première génération.

### Résumé de l'Invention

La présente invention concerne des souches de levure *Saccharomyces cerevisiae* qui présentent des propriétés améliorées par rapport aux souches de levure spécialisées couramment utilisées dans la production d'éthanol de première génération.

Ainsi, en particulier, la présente invention a pour objet la souche de levure *Saccharomyces cerevisiae* 53-137 qui a été déposée le 25 juillet 2013 à la CNCM (Collection Nationale de Cultures de Micro-organismes de l'Institut Pasteur, 25 rue du Docteur Roux, 75724 Paris, Cedex 15) sous le numéro 1-4791 dans les conditions du traité de Budapest.

La présente invention a aussi pour objet la souche de levure *Saccharomyces cerevisiae* 53-005 qui a été déposée le 25 juillet 2013 à la CNCM (Collection Nationale de Cultures de Micro-organismes de l'Institut Pasteur, 25 rue du Docteur Roux, 75724 Paris, Cedex 15) sous le numéro 1-4790 dans les conditions du traité de Budapest.

La présente invention a également pour objet la souche de levure *Saccharomyces cerevisiae* 53-214 qui a été déposée le 25 juillet 2013 à la CNCM (Collection Nationale de Cultures de Micro-organismes de l'Institut Pasteur, 25 rue du Docteur Roux, 75724 Paris, Cedex 15) sous le numéro 1-4792 dans les conditions du traité de Budapest.

La présente invention concerne également une levure obtenue par culture d'une souche de levure *Saccharomyces cerevisiae* choisie parmi la souche de levure *Saccharomyces* qui a été déposée le 25 juillet 2013 à la CNCM sous le numéro 1-4791, la souche de levure *Saccharomyces cerevisiae* qui a été déposée le 25 juillet 2013 à la CNCM sous le numéro 1-4790 et la souche de levure *Saccharomyces cerevisiae* qui a été déposée le 25 juillet 2013 à la CNCM sous le numéro 1-4792.

La présente invention a également pour objet l'utilisation d'une souche de levure *Saccharomyces cerevisiae* choisie parmi la souche de levure *Saccharomyces* qui a été déposée le 25 juillet 2013 à la CNCM sous le numéro 1-4791, la souche de levure *Saccharomyces cerevisiae* qui a été déposée le 25 juillet 2013 à la CNCM sous le numéro 1-4790 et la souche de levure *Saccharomyces cerevisiae* qui a été déposée le 25 juillet 2013 à la CNCM sous le numéro 1-4792 ou d'une levure obtenue par culture de l'une de ces souches, pour la production d'éthanol de première génération à partir de biomasse.

La présente invention a encore pour objet un procédé de production d'éthanol de première génération à partir de biomasse comprenant une étape de fermentation utilisant une souche de levure *Saccharomyces cerevisiae* choisie parmi la souche de levure *Saccharomyces* qui a été déposée le 25 juillet 2013 à la CNCM sous le numéro 1-4791, la souche de levure *Saccharomyces cerevisiae* qui a été déposée le 25 juillet 2013 à la CNCM sous le numéro 1-4790 et la souche de levure *Saccharomyces cerevisiae* qui a été déposée le 25 juillet 2013 à la CNCM sous le numéro 1-4792 ou une levure obtenue par culture de l'un de ces souches.

Dans certains modes de réalisation, la biomasse est riche en sucre et/ou amidon et est choisie parmi, ou provient notamment du maïs, du blé, de l'orge, du seigle, du sorgho, du manioc, du triticale, de la pomme de terre, de la patate douce, de la canne à sucre, de la betterave sucrière, du sorgho sucrier.

Dans certains modes de réalisation préférés, la biomasse est choisie parmi ou provient du maïs, du blé, de l'orge, du manioc, de la betterave sucrière, de la canne à sucre.

La présente invention a encore pour objet la fabrication des drèches et drèches additionnées de solubles des résidus de fermentation obtenus au cours des procédés de production d'éthanol.

Une description plus détaillée de certains modes de réalisation préférés de l'invention est donnée ci-dessous.

### Description Détaillée de l'Invention

Comme mentionné plus haut, la présente invention concerne trois souches de levure *Saccharomyces cerevisiae* utiles pour la production d'éthanol de première génération. Les trois souches de l'invention ont été obtenues par un programme d'hybridation et de sélection. Elles résultent de l'hybridation entre la souche de levure *Saccharomyces cerevisiae* déposée à la CNCM le 4 septembre 2008 sous le numéro 1-4072, une souche appartenant au Déposant, et décrite dans WO2010/031916 et le clone de levure *Saccharomyces cerevisiae* déposé à la CNCM le 26 juin 2013 sous le numéro 1-4782, qui appartient également au Déposant.

La souche de levure 1-4072 est une souche sélectionnée par le Déposant comme ayant la plus forte tolérance à l'éthanol parmi un panel de 21 souches évaluées.

Le clone de levure *Saccharomyces cerevisiae* 1-4782 a été sélectionné par le Déposant comme ayant une tolérance à l'éthanol élevée quoique inférieure à celle de la souche 1-4072 mais comme ayant une production de glycérol inférieure à celle de la souche 1-4072.

Le programme de sélection des hybrides a conduit aux trois souches de l'invention. Chacune de ces souches constitue une alternative aux souches spécialisées les plus performantes qui sont aujourd'hui utilisées dans la production industrielle d'éthanol de première génération. En effet, les trois souches de l'invention présentent une plus forte tolérance à l'éthanol que la souche de référence 1-4072 et en même temps produisent de l'éthanol de première génération avec un rendement plus élevé que la souche 1-4072 du fait d'une plus faible production de glycérol, qui est un sous-produit de la réaction de fermentation. Cette bonne tolérance est observée quelle que soit la température (32°C, 35°C ou 38°C), le pH (4,0, 5,0 ou 5,5) et l'apport d'azote minéral (150 à 500 ppm) pendant la fermentation, ce qui en fait des souches particulièrement adaptées à la production d'éthanol de première génération où des fluctuations de paramètres de fermentation sont courantes. De plus, les souches de l'invention présentent l'avantage d'avoir une cinétique de production d'éthanol qui est similaire ou légèrement inférieure à celle de la souche de référence 1-4072.

L'invention concerne également une levure obtenue par culture d'une des souches de l'invention. Les procédés de culture d'une souche de levure sont connus dans l'art, et l'homme du métier sait optimiser les conditions de culture pour chaque souche en fonction de sa nature.

Les souches de levure de l'invention et les levures obtenues par culture de ces souches trouvent application dans la production d'éthanol de première génération à partir de biomasse. On entend ici par « biomasse », l'ensemble des matières organiques d'origine végétale pouvant devenir source d'énergie après transformation. Préférablement, dans le contexte de l'invention, la biomasse est issue des produits et/ou coproduits agricoles ou agro-alimentaires. En particulier, la biomasse est préférablement riche en saccharose ou en amidon, et est choisie parmi, ou est issue de, par exemple, le maïs, le blé, l'orge, le seigle, le sorgho, le manioc, le triticale, la pomme de terre, la patate douce, la canne à sucre, la betterave sucrière, le sorgho sucrier.

Dans certains modes de réalisation préférés, la biomasse est choisie parmi, ou est issue du maïs, du blé, de l'orge, ou du manioc.

Les procédés de production d'éthanol de première génération à partir de biomasse et l'utilisation de levures dans l'étape de fermentation sont connus dans l'art. Le procédé industriel le plus commun recourt à des traitements physique, chimique et biochimique qui visent ultimement à permettre la fermentation de sucres et à produire l'éthanol. Plusieurs variantes de ce procédé existent et sont connus de l'homme du métier. Les souches de levure de l'invention et les levures obtenues par culture de ces souches peuvent être employées dans n'importe quelle méthode de production d'éthanol de première génération.

L'invention s'applique particulièrement à la production d'éthanol en tant que carburant, mais aussi à la production d'éthanol pour les industries alimentaires, chimiques, pharmaceutiques et cosmétiques.

A moins qu'ils ne soient définis d'une autre manière, tous les termes techniques et scientifiques utilisés dans la description ont la même signification que celle couramment comprise par un spécialiste ordinaire du domaine auquel appartient cette invention. De même, toutes les publications, demandes de brevet, tous les brevets et toutes autres références mentionnés ici sont incorporés par référence.

### Exemples

Les exemples suivants décrivent certains modes de réalisation de la présente invention. Cependant, il est entendu que les exemples et les figures ne sont présentés qu'à titre illustratif et ne limitent en aucun cas la portée de l'invention.

### Légende des Figures

**Figure 1****:** Concentrations finales en fin des essais de fermentation réalisées sur milieux de fermentation liquéfiés selon l'Exemple 1. **(A)** Concentration en éthanol et **(B)** concentration en glycérol. Les milieux A, B et C sont décrits dans le tableau 1.
**Figure 2** **:** Cinétique de production d'éthanol par la souche 1-4072 **(A)** et les hybrides 1-4790 **(B),** 1-4791 **(C)** et 1-4792 **(D)** dans différentes conditions de fermentation.

### Exemple 1 : Sélection des Souches Parentales à Croiser avec la Souche 1-4072

Un panel de 21 souches ou clones a été testé en fermentation alcoolique. Les essais ont été réalisés dans des conditions de saccharification et fermentation simultanées (SSF) avec un excès de sucres par rapport aux capacités de conversion en éthanol des souches. Dans un premier temps, des milieux synthétiques, contenant des dextrines d'amidon avec ajout ou non de solubles des résidus de distillation ont été utilisés. Dans un second temps, des milieux de fermentation préparés à partir de farine de maïs, de fractions des solubles, des résidus de distillation et d'eau de procédé dans les proportions données dans le tableau ci-dessous ont été utilisés. Ces différents éléments ont été obtenus à partir d'une usine de production d'éthanol industriel selon un procédé de première génération. Le mélange à base de farine de maïs a été liquéfié à l'aide de Liquozyme^{™} SCDS (Novozymes) à 85°C pendant 3 heures après ajustement du pH à 5,6. La dose d'emploi de Liquozyme^{™} a été de 0.8 mL/kg de farine mise en oeuvre. Avant ensemencement, le pH initial a été ajusté à 5,0 ou 4,5 ; différentes concentrations en urée (300, 600 et 1000 ppm d'équivalent azote) ont été ajoutées comme indiqué dans le tableau suivant. Spyrizyme Fuel Ultra^{™} (Novozymes) a été ajouté à la dose de 0,6 mL/kg de farine. La température de fermentation a été régulée à 32°C. Les souches ont été préalablement propagées dans un milieu synthétique riche dans des flacons à baffles de 500 mL (propagation micro-aérée).

**Tableau 1.**

| **Milieu** | **A** | **B** | **C** |
|---|---|---|---|
| **pH initial** | **pH 4,5** | **pH 5,0** | **pH 4,5** |
| Température (°C) | 32 | 32 | 32 |
| Farine (g) | 362,5 | 362,5 | 362,5 |
| Solubles de résidus de distillation (g) | 300 | 250 | 300 |
| Urée (ppm) | 300 | 600 | 1000 |
| Eau de procédé | QSP 1 kg | QSP 1 kg | QSP 1 kg |

Parmi les différentes souches testées, la souche déposée à la CNCM sous le numéro 1-4072 a été retenue comme étant la souche produisant le plus d'éthanol des souches testées. Parmi les différentes souches testées, la souche 1-4782 a été retenue comme étant une souche produisant de grandes quantités d'éthanol quoique inférieures à 1-4072 et comme produisant de moins grandes quantités de glycérol que la souche 1-4072.

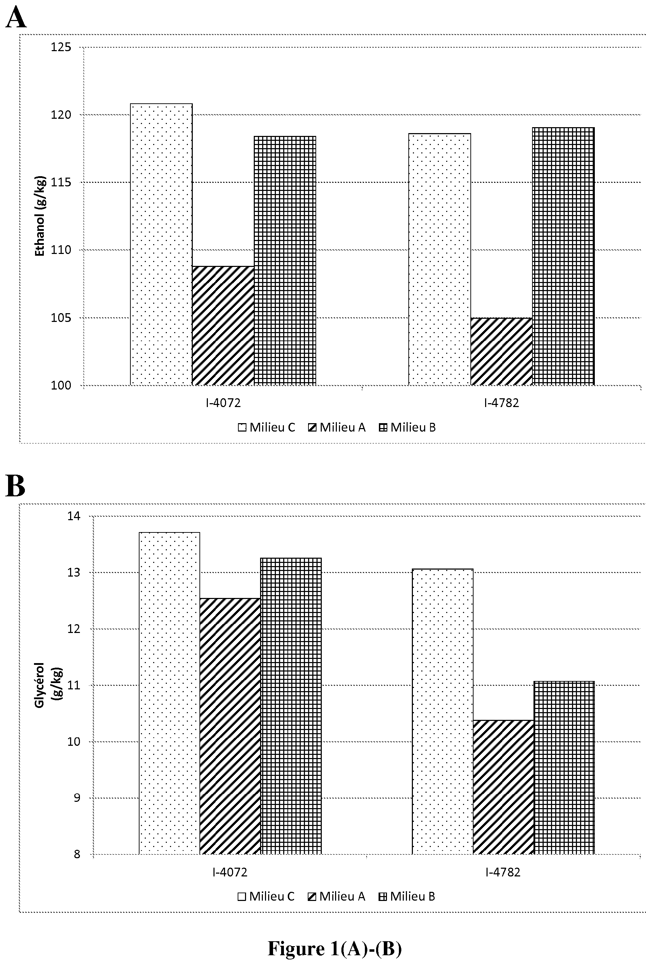

Il est intéressant de noter que la cinétique de production d'éthanol du clone 1-4782 est assez lente par rapport à celle de la souche 1-4072. Les concentrations finales en éthanol et en glycérol obtenues au cours des essais sont montrées dans les figures 1A et 1B.

L'objectif était donc d'obtenir, par hybridation, au moins un hybride ayant une tolérance à l'éthanol supérieure à celle de la souche 1-4072, une production en glycérol moindre que celle de la souche 1-4072, et une cinétique de production en éthanol au moins similaire à celle de la souche 1-4072.

### Exemple 2 : Obtention de Nouveaux Hybrides

Une fois les souches parentales sélectionnées, différentes étapes techniques conduisant à la création de nouvelles souches par croisement ont été effectuées comprenant :
- l'obtention par sporulation puis germination de ségrégeants des deux souches parentes et caractérisation de leur type sexuel,
- la réalisation des croisements après établissement de tableaux de croisement, et
- l'isolement et l'identification des nouveaux hybrides.

***Sélection des ségrégeants élites de la souche I-4072.*** Des ségrégeants de la souche 1-4072 ont été générés et évalués sur la base de leur production d'éthanol. Puis, sur la base de ces résultats, 8 spores de type sexuel « alpha » et 12 spores de type sexuel « a » possédant les meilleures performances alcool ont été sélectionnés.

***Réalisation des croisements et obtention des nouvelles souches.*** Afin d'obtenir de nouvelles souches, plusieurs séries de croisements ont été réalisées entre les ségrégeants de 1-4072 sélectionnés et des ségrégeants de 1-4782 choisis au hasard.

***Identification des nouveaux hybrides.*** Les nouveaux hybrides ont été identifiés par PCR mating type.

De nombreuses souches ont ainsi été créées et validés à l'issue des croisements réalisés. Parmi les hybrides 1-4791, 1-4790 et 1-4792, objets de la présente demande, se distinguent particulièrement, comme indiqué plus loin.

### Exemple 3 : Sélection des Nouveaux Hybrides

Afin de pouvoir sélectionner les meilleurs hybrides parmi les 292 souches nouvellement créées, 4 niveaux de sélection ont été développés. Ces sélections sont basées sur des suivis de perte de masse de milieux de fermentation alcool après ensemencement avec les souches à étudier contre le témoin 1-4072 et une mesure de la concentration de l'éthanol, du glucose restant et du glycérol produit après 72 heures de fermentation à différentes températures (35°C et 38°C). Les milieux de fermentation utilisés ont été des milieux synthétiques contenant une concentration en glucose élevée, supérieure aux capacités de conversion en éthanol des souches testées. Les critères de sélection sont une meilleure cinétique de perte de masse que la souche 1-4072 et/ou une production de glycérol diminuée par rapport à la souche 1-4072. La perte de masse est un indicateur indirect de la production d'éthanol par les levures selon l'équation stoechiométrique : 1 mol glucose → 2 mol CO₂ + 2 mol éthanol qui permet de relier globalement la masse de perte de masse du milieu sous forme de CO₂ produit et évaporé à la masse d'éthanol produit.

Au final, sur 292 souches, 18 hybrides ont été sélectionnés, dont les souches 1-4790, 1-4791 et 1-4792. Ces dernières ont été choisies car elles présentent les caractéristiques suivantes :
- hybride 1-4790 : aux températures de 35°C et 38°C, pour une production d'éthanol équivalente à celle de la souche 1-4072, la production en glycérol est diminuée de 15 à 20%. La cinétique de perte de masse est cependant inférieure à celle de la souche 1-4072.
- hybride 1-4791 : aux températures de 35°C et 38°C, la production d'éthanol est augmentée de 3,5% par rapport à celle de la souche 1-4072, la cinétique de perte de masse est plus rapide que celle de la souche 1-4072, et la production de glycérol est équivalente à celle de la souche 1-4072.
- hybride I-4792 : aux températures de 35°C et 38°C, la production d'éthanol est équivalente à celle de 1-4072 (avec une légère amélioration à 38°C), la cinétique de perte de masse est équivalente à celle de 1-4072, et la production de glycérol est inférieure de 7,5 à 8% par rapport à celle de 1-4072.

### Exemple 4 : Production d'Ethanol à partir de Farine de Maïs s par les Hybrides en excès de sucres dans le milieu

Des tests ont été effectués dans des milieux à base de farine de maïs et de fraction des solubles des résidus de distillation industriels. Les essais ont été effectués à différentes températures de fermentation, avec différents ajouts d'azote et à différents pH initiaux. Les caractéristiques qui ont été étudiées sont : la tolérance maximale des souches à l'éthanol, la production de glycérol et la cinétique de production d'éthanol.

Les pertes de masse des milieux de fermentation ont été mesurées au cours du temps. A stabilisation de la perte de masse, un prélèvement du milieu de fermentation a été réalisé et un dosage par HPLC des concentrations en éthanol et glycérol a été réalisé. Les masses d'éthanol et glycérol produites ont été calculées à partir de la concentration mesurée et de la masse de moût de fermentation au moment du prélèvement pour effectuer le dosage et des valeurs de concentration et de masses initiales.

***Protocole.*** Les milieux de fermentation ont été préparés à partir de farine de maïs, de fractions des solubles des résidus de distillation et d'eau de procédé. Afin de simuler au mieux les conditions de production industrielle, ces différents éléments ont été obtenus à partir d'usines de production d'éthanol industriel selon des procédés de première génération. Les proportions des trois composants industriels étaient : farine de maïs (36% w/w), fraction des solubles des résidus de distillation (35% w/w) et l'eau de procédé (29% w/w). Le mélange a été liquéfié à l'aide de Liquozyme^{™} SCDS (Novozymes) à 85°C pendant 3 heures après ajustement du pH à 5,6. La dose d'emploi de Liquozyme^{™} a été de 0,8 mL/kg de farine mise en oeuvre. Avant ensemencement, le pH a été ajusté à 5 ou 4 (selon tableau), différentes concentrations en urée (150, 250 et 500 ppm d'équivalent azote) ont été ajoutées. Spyrizyme Fuel Ultra^{™} (Novozymes) a été ajouté à la dose de 0,6 mL/kg de farine. Les souches ont été préalablement propagées dans un milieu synthétique riche dans des flacons à baffles de 500 mL (propagation micro-aérée). Une crème de levure a été préparée à partir du milieu de propagation par centrifugation et remise en suspension dans de l'eau du culot de centrifugation. Une matière sèche a été réalisée sur la crème de levure et le milieu de fermentation a été ensemencé par la crème de façon à avoir un taux d'ensemencement de 0,5 g d'équivalent levure sèche/kg de milieu.

***Tests effectués.*** Le tableau suivant présente les conditions des essais de fermentation effectués et des souches utilisées.

**Tableau 2. Conditions des essais de fermentation.**

| Essais | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| Conditions | | | | | | | | | | | | | | | |
| Milieu | A | A | A | A | A | A | B | B | B | B | B | B | B | B | B |
| T (°C) | 32 | 35 | 38 | 35 | 35 | 35 | 35 | 35 | 35 | 32 | 32 | 35 | 35 | 38 | 38 |
| N (ppm) | 500 | 500 | 500 | 150 | 250 | 500 | 150 | 250 | 500 | 500 | 500 | 500 | 500 | 500 | 500 |
| pH | 5 | 5 | 5 | 5 | 5 | 5 | 5 | 5 | 5 | 5 | 4 | 5 | 4 | 5 | 4 |

| Souches testées | | | | | | | | | | | | | | | |
|---|---|---|---|---|---|---|---|---|---|---|---|---|---|---|---|
| I-4072 | x | x | x | x | x | x | x | x | x | x | x | x | x | x | x |
| I-4790 | x | x | x | x | x | x | x | x | x | x | x | x | x | x | x |
| I-4791 | x | x | x | x | x | x | x | x | x | x | x | x | x | x | x |
| I-4792 | | | | | | x | | | x | x | x | x | x | x | x |

| | | | | | | | | | | | | | | | |
|---|---|---|---|---|---|---|---|---|---|---|---|---|---|---|---|
| A: 36% farine de maïs, 35% solubles des résidus de distillation, 29% d'eau de procédé B: 36% farine de maïs, 35% solubles des résidus de distillation, 29% d'eau de procédé N : Apport d'azote | | | | | | | | | | | | | | | |

***Résultats.*** Les résultats obtenus sont présentés dans les tableaux ci-dessous.

Tableaux 3 à 4. Concentrations finales en éthanol et en glycérol et masses d'éthanol et de glycérol produites dans les essais 1-9.

**Tableau 3.**

| Souche | Concentrations finales (g/kg) | | | Masse produite (g/kg de milieu mis en oeuvre) | |
|---|---|---|---|---|---|
| | Equiv.glucose | glycérol | Ethanol | glycérol | Ethanol |
| **Essai 1 :** | **32°C, 500 ppm N, pH5** | | | | |
| **I-4072** | **40,9** | **7,9** | **121,3** | **5,3** | **107,6** |
| I-4790 | 35,4 | 7,5 | 123,9 | 4,8 | 109,3 |
| I-4791 | 37,1 | 8,4 | 129,6 | 5,7 | 114,8 |
| **Essai 2 :** | **35°C, 500 ppm N, pH5** | | | | |
| **I-4072** | **37,7** | **6,7** | **110,5** | **5,0** | **98,9** |
| I-4790 | 28,7 | 6,2 | 116,4 | 4,5 | 103,8 |
| I-4791 | 32,7 | 6,5 | 111,5 | 4,8 | 99,7 |
| **Essai 3 :** | **38°C, 500 ppm N, pH5** | | | | |
| **I-4072** | **75,6** | **6,4** | **90,3** | **4,8** | **82,3** |
| I-4790 | 74,0 | 5,5 | 93,5 | 4,0 | 85,2 |
| I-4791 | 72,1 | 6,3 | 93,4 | 4,6 | 85,0 |
| **Essai 4 :** | **150 ppm N, 35°C, pH5** | | | | |
| **I-4072** | **46,2** | **15,9** | **109,9** | **8,5** | **98,6** |
| I-4790 | 26,6 | 14,8 | 116,1 | 7,4 | 103,4 |
| I-4791 | 34,4 | 15,5 | 113,5 | 8,1 | 101,3 |
| **Essai 5 :** | **250 ppm N, 35°C, pH5** | | | | |
| **I-4072** | **27,9** | **15,9** | **108,2** | **8,5** | **96,4** |
| I-4790 | 20,5 | 15,3 | 117,7 | 7,9 | 104,4 |
| I-4791 | 26,6 | 15,3 | 112,3 | 8,0 | 99,8 |
| **Essai 6 :** | **500 ppm N, 35°C, pH5** | | | | |
| **I-4072** | **15,9** | **15,7** | **117,4** | **8,3** | **104,2** |
| I-4790 | 11,4 | 14,8 | 122,0 | 7,4 | 108,0 |
| I-4791 | 16,0 | 15,0 | 119,9 | 7,7 | 106,4 |
| I-4792 | 11,2 | 15,4 | 122,4 | 8,0 | 108,4 |
| **Essai 7 :** | **150 ppm N, 35°C, pH5** | | | | |
| **I-4072** | **27,8** | **15,9** | **103,9** | **9,0** | **93,4** |
| I-4790 | 17,1 | 15,2 | 111,1 | 7,9 | 99,5 |
| I-4791 | 23,1 | 15,9 | 108,2 | 8,6 | 97,1 |
| **Essai 8 :** | **250 ppm N, 35°C, pH5** | | | | |
| **I-4072** | **35,2** | **16,3** | **114,9** | **9,0** | **102,6** |
| I-4790 | 22,6 | 15,5 | 122,1 | 8,1 | 108,4 |
| I-4791 | 30,6 | 15,2 | 117,5 | 7,9 | 104,6 |
| **Essai 9 :** | **500 ppm N, 35°C, pH5** | | | | |
| **I-4072** | **28,4** | **15,8** | **123,2** | **8,2** | **109,2** |
| I-4790 | 18,6 | 15,3 | 128,1 | 7,7 | 113,3 |
| I-4791 | 22,5 | 14,8 | 122,5 | 7,4 | 108,6 |
| I-4792 | 17,3 | 15,1 | 125,5 | 7,6 | 111,0 |

**Tableau 4.**

| Souche | Concentrations finales (g/kg) | | | Masse produite (g/kg de milieu mis en oeuvre) | |
|---|---|---|---|---|---|
| | Equiv.glucose | glycérol | Ethanol | glycérol | Ethanol |
| **Essai 11 :** | **32°C, 500 ppm N, pH4** | | | | |
| **I-4072** | **35,4** | **15,0** | **117,1** | **13,4** | **104,2** |
| I-4790 | 27,0 | 14,4 | 122,9 | 12,8 | 109,0 |
| I-4791 | 35,5 | 14,6 | 117,7 | 13,0 | 104,9 |
| I-4792 | 26,9 | 14,4 | 122,9 | 12,7 | 108,9 |
| **Essai 10 :** | **32°C, 500 ppm N, pH5** | | | | |
| **I-4072** | **18,5** | **16,2** | **127,3** | **14,3** | **112,2** |
| I-4790 | 16,2 | 15,1 | 129,5 | 13,3 | 114,1 |
| I-4791 | 22,5 | 15,5 | 126,2 | 13,7 | 111,3 |
| I-4792 | 15,3 | 15,3 | 130,3 | 13,4 | 114,6 |
| **Essai 13 :** | **35°C, 500 ppm N, pH4** | | | | |
| **I-4072** | **46,4** | **15,1** | **105,5** | **13,6** | **94,7** |
| I-4790 | 33,8 | 14,5 | 117,6 | 12,9 | 104,7 |
| I-4791 | 46,0 | 14,8 | 110,6 | 13,1 | 98,5 |
| I-4792 | 37,3 | 14,9 | 114,8 | 13,3 | 102,4 |
| **Essai 12 :** | **35°C, 500 ppm N, pH5** | | | | |
| **I-4072** | **31,6** | **16,5** | **117,4** | **14,7** | **1044** |
| I-4790 | 30,5 | 15,4 | 119,9 | 13,7 | 106,6 |
| I-4791 | 31,7 | 15,7 | 117,7 | 14,0 | 104,7 |
| I-4792 | 27,2 | 15,9 | 121,2 | 14,1 | 107,6 |
| **Essai 15 :** | **38°C, 500 ppm N, pH4** | | | | |
| **I-4072** | **82,7** | **14,1** | **87,7** | **12,9** | **80,2** |
| I-4790 | 80,3 | 13,1 | 85,8 | 11,9 | 78,4 |
| I-4791 | 80,9 | 12,9 | 88,1 | 11,8 | 80,5 |
| I-4792 | 82,7 | 13,5 | 86,4 | 123 | 78,9 |
| **Essai 14 :** | **38°C, 500 ppm N, pH5** | | | | |
| **I-4072** | **72,7** | **14,8** | **93,4** | **13,5** | **84,8** |
| I-4790 | 76,0 | 13,8 | 92,2 | 12,5 | 83,9 |
| I-4791 | 76,8 | 14,3 | 92,1 | 13,0 | 83,8 |
| I-4792 | 75,8 | 14,070 | 93,375 | 12,8 | 84,9 |

Ces résultats montrent clairement que les hybrides ont une meilleure tolérance à l'éthanol que la souche de départ I-4072 (présentant un avantage de 2 à 3% sur la souche I-4072) et qu'ils produisent moins de glycérol que la souche I-4072. Cette moindre production de glycérol par les souches I-4790, I-4791 et I-4792 devrait logiquement entraîner un meilleur rendement de conversion des sucres consommés en éthanol.

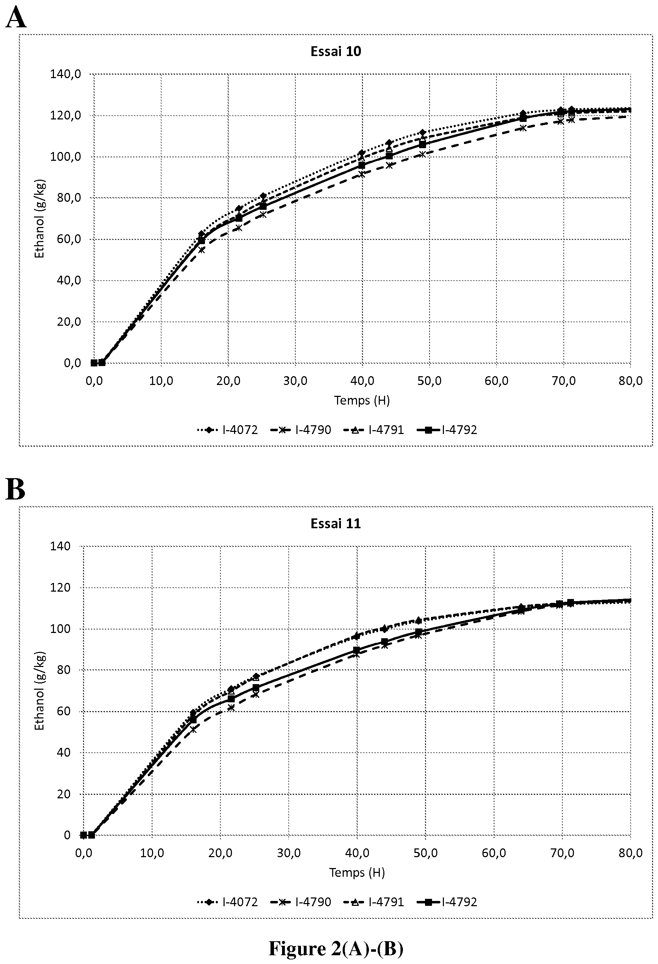

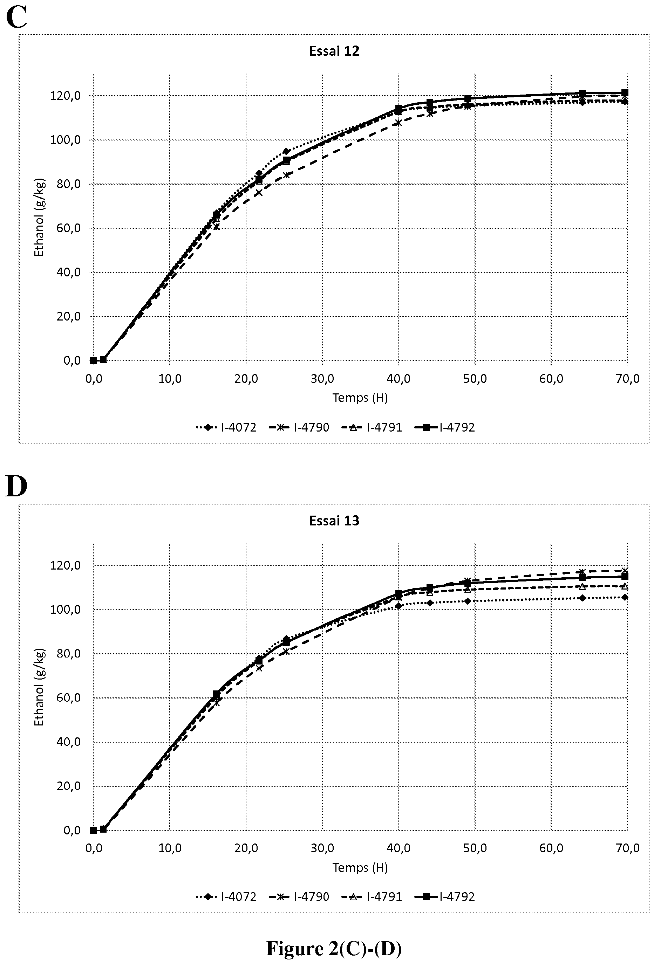

***Cinétique de Production d'Ethanol.*** A titre d'exemple, les cinétiques de perte de masse des essais des essais 10-13 sont présentées sur la Figure 2.

Ces résultats montrent que la souche I-4072 possède la cinétique de production d'éthanol la plus rapide de toutes les souches testées. Parmi les hybrides de l'invention, l'hybride I-4791 a une cinétique de production d'éthanol qui est à peu près similaire à celle de la souche I-4072. Par contre, l'hybride I-4790 présente la cinétique la plus lente avec des retards allant jusqu'à 10 heures à pH 5.

### Exemple 5 : Tests de Production d'Ethanol à partir de Farine de Maïs par les Hybrides en Concentrations Limitantes en Sucres dans le Milieu

***Rendement de Conversion en Ethanol.*** Afin de conforter les résultats précédents, plusieurs essais ont été conduits avec des concentrations en farines de maïs inférieures à celles utilisées dans les essais de l'Exemple 4 et telles que les souches pourront consommer totalement les sucres fermentescibles du milieu. Les essais réalisés sont donnés dans le tableau 5 suivant :

**Tableau 5. Conditions de fermentation**

| **Essai** | **16** | **17** | **18** | **19** |
|---|---|---|---|---|
| **Conditions** | | | | |
| Milieu | C | D | C | D |
| T(°C) | 32 | 35 | 32 | 35 |
| N (ppm) | 250 | 250 | 150 | 150 |
| pH | 5 | 5 | 5 | 5 |

| **Souches testées** | | | | |
|---|---|---|---|---|
| I-4072 | x | x | x | x |
| I-4790 | x | x | x | x |
| I-4791 | x | x | x | x |
| I-4792 | x | x | x | x |

| | | | | |
|---|---|---|---|---|
| C: 32°C: 33% farine de maïs, 35% solubles des résidus de distillation, 32% d'eau de procédé. D: 35°C: 30% farine de maïs, 35% solubles des résidus de distillation, 35% d'eau de procédé. | | | | |

Les résultats sont présentés dans les tableaux 6 et 7suivants.

**Tableau 6.**

| Souche | Concentrations finales (g/kg) | | | Masse produite (g/kg de milieu mis en oeuvre) | |
|---|---|---|---|---|---|
| | Equiv. glucose | glycérol | Ethanol | glycérol | Ethanol |
| **Essai 16** | **32°C, 150 ppm N, pH5** | | | | |
| I-4072 | 1,6 | 16,9 | 118,7 | 8,3 | 105,3 |
| I-4790 | 1,6 | 15,9 | 122,3 | 7,4 | 108,6 |
| I-4791 | 1,8 | 17,1 | 121,9 | 8,5 | 108,2 |
| I-4792 | 1,9 | 16,5 | 119,8 | 7,9 | 106,4 |
| **Essai 17** | **35°C, 150 ppm N, pH5** | | | | |
| I-4072 | 1,5 | 16,1 | 102,4 | 7,7 | 91,7 |
| I-4790 | 1,4 | 14,7 | 107,9 | 6,5 | 96,6 |
| I-4791 | 1,5 | 15,1 | 108,1 | 6,8 | 96,6 |
| I-4792 | 1,4 | 15,0 | 109,6 | 6,7 | 97,9 |

**Tableau 7.**

| Souche | Concentrations finales (g/kg) | | | Masse produite (g/kg de milieu mis en oeuvre) | |
|---|---|---|---|---|---|
| | Equiv. glucose | glycérol | Ethanol | glycérol | Ethanol |
| **Essai 18** | **32°C, 250 ppm N, pH5** | | | | |
| I-4072 | 1,9 | 14,8 | 116,2 | 8,1 | 103,6 |
| I-4790 | 1,5 | 13,9 | 117,1 | 7,2 | 104,4 |
| I-4791 | 1,7 | 13,9 | 117,3 | 7,3 | 104,7 |
| I-4792 | 1,6 | 14,6 | 116,7 | 7,8 | 104,2 |
| **Essai 19** | **35°C, 250 ppm N, pH5** | | | | |
| I-4072 | 1,5 | 14,5 | 106,1 | 7,9 | 95,3 |
| I-4790 | 1,4 | 13,0 | 106,5 | 6,5 | 95,9 |
| I-4791 | 1,5 | 13,6 | 105,8 | 7,1 | 95,2 |
| I-4792 | 1,5 | 13,9 | 103,2 | 7,3 | 92,9 |

Les résultats obtenus confirment les observations faites dans les tests précédents: les hybrides 1-4790, 1-4791 et 1-4792 produisent significativement moins de glycérol et plus d'éthanol que la souche de référence 1-4072 en particulier à dose d'azote (N) apporté réduit.

***Conclusions.*** Les résultats obtenus démontrent clairement que:
- l'hybride 1-4791 présente : une cinétique de production d'éthanol identique ou similaire à celle de 1-4072, une meilleure tolérance à l'éthanol (+2%), une production moindre de glycérol (-5%), et un rendement meilleur en éthanol (+2%) ;
- l'hybride 1-4790 présente : une meilleure tolérance à l'éthanol que 1-4072 (+3%), une production moindre de glycérol (-10%) et un meilleur rendement en éthanol (+2%) mais une cinétique de production d'éthanol plus lente que celle de 1-4072.
- l'hybride 1-4792 présente : une meilleure tolérance à l'éthanol que 1-4072 (+3%) sauf dans des conditions rudes (38°C/pH 4), une cinétique de production d'éthanol similaire à celle de 1-4072, une production moindre de glycérol (-5%) et un rendement plus élevé en éthanol (+1%).

### Exemple 6 : Essais de Production de Levures à partir des 3 Hybrides Sélectionnés

Les hybrides sélectionnés ont été multipliés, à l'échelle pilote, en conditions aérées suivant un schéma fed-batch bien connu de l'homme du métier. Les levures obtenues ont été séchées selon les techniques habituelles. Ces essais de production de levures se sont déroulés sans qu'aucun problème particulier ne soit constaté.

Des essais de production d'éthanol ont été réalisés à partir des levures sèches instantanées produites dans le but de vérifier que le procédé de production de la levure n'altère pas les performances des souches produites.

***Protocole.*** Les milieux de fermentation ont été préparés à partir de farine de maïs, de fractions des solubles des résidus de distillation et d'eau de procédé. Ces différents éléments ont été obtenus d'usines de production d'éthanol industriel. Les proportions des trois composants industriels étaient : farine de maïs (36% w/w), fraction des solubles de distillation (35% w/w) et eau (29% w/w). Le mélange a été liquéfié à l'aide de Liquozyme SCDS^{™} (Novozymes) à 85°C pendant 3 heures après ajustement du pH à 5,6. La dose d'emploi de Liquozyme^{™} a été de 0,8 mL/kg de farine mise en oeuvre.

Les levures sèches produites à partir des hybrides I-4790, I-4791 et I-4792 et de la levure commerciale Ethanol Red^{™} ont été propagées sur le milieu liquéfié dilué dans de l'eau (70% m/m, 30% m/m). Le taux d'ensemencement était de 0,5 g de levure sèche/kg de milieu, le pH a été ajusté à 5, la température de propagation a été de 32°C, de l'urée (500 ppm) et Spirizyme Fuel Ultra^{™} (0,6 mL/kg de farine) ont été ajoutés. Le milieu de propagation a été transféré au milieu de fermentation avec un transfert de 10% masse/masse.

Les fermentations ont été réalisées dans les conditions décrites dans le tableau 8.

**Tableau 8.**

| **Essais** | | **20** | **21** | **22** | **23** |
|---|---|---|---|---|---|
| **Conditions** | | | | | |
| Milieu | | B | B | B | B |
| T (°C) | | 32 | 35 | 32 | 35 |
| urée (ppm) | | 500 | 500 | 300 | 300 |
| pH initial | | 5 | 5 | 5 | 5 |

| **levures sèches instantanées testées** | | | | | |
|---|---|---|---|---|---|
| levure Ethanol Red™ | | x | x | x | x |
| I-4790 | Lot 1 | x | x | x | x |
| I-4790 | Lot 2 | x | x | x | X |
| I-4791 | Lot 1 | x | x | x | X |
| I-4791 | Lot 2 | X | x | x | x |
| I-4792 | Lot 1 | X | x | x | x |
| I-4792 | Lot 2 | x | x | x | X |

| | | | | | |
|---|---|---|---|---|---|
| B: 36% farine de maïs, 35% de solubles issus des résidus de distillation, 29% d'eau de procédé | | | | | |

Les résultats obtenus, qui sont donnés dans le tableau 9, confirment dans la limite de la précision des essais réalisés les résultats déjà obtenus et confirment la possibilité de produire et sécher les souches I-4790, I-4791 et I-4792.

***Conclusions.*** Les résultats obtenus selon l'invention ont permis d'obtenir des levures industrielles ayant des performances significativement améliorées en termes de productivité et de rendement lors de leur mise en oeuvre pour la production d'éthanol à partir de sucres issus de biomasses végétales.

Par rapport au produit de référence du marché (Ethanol Red^{™}) :
- La souche industrielle 1-4790 permet d'améliorer la productivité en éthanol de +2% avec un rendement éthanol/sucre amélioré grâce à la réduction de 10% de la quantité de glycérol générée ;
- La souche industrielle 1-4791 permet d'améliorer la productivité en éthanol de +2% avec un rendement éthanol/sucre amélioré grâce à la réduction de 5% de la quantité de glycérol générée ;
- La souche industrielle I-4792 permet d'améliorer la productivité en éthanol de +1% avec un rendement éthanol/sucre amélioré grâce à la réduction de 5% de la quantité de glycérol générée.

**Tableau 9.**

| | | concentrations finales (g/kg) | | | Masses produites (g/kg de milieu mis en oeuvre) | |
|---|---|---|---|---|---|---|
| Levure sèche instantanée | lot | glucose | glycérol | éthanol | glycérol | éthanol |
| 32°C, 300 ppm | | | | | | |
| Ethanol Red™ | | 18,3 | 16,9 | 113,5 | 8,3 | 95,6 |
| I-4791 | 1 | 10,6 | 16,8 | 118,1 | 8,1 | 99,2 |
| I-4791 | 2 | 10,7 | 16,8 | 119,5 | 8,1 | 100,5 |
| I-4790 | 1 | 12,1 | 15,8 | 116,8 | 7,3 | 98,4 |
| I-4790 | 2 | 16,5 | 16,1 | 109,5 | 7,7 | 92,2 |
| I-4792 | 1 | 16,2 | 16,4 | 112,9 | 7,8 | 95,2 |
| I-4792 | 2 | 14,4 | 16,4 | 112,8 | 7,8 | 94,8 |

| 35°C, 300 ppm urée | | | | | | |
|---|---|---|---|---|---|---|
| Ethanol Red™ | | 33,6 | 16,1 | 103,1 | 7,7 | 86,9 |
| I-4791 | 1 | 29,7 | 15,9 | 103,5 | 7,4 | 87,0 |
| I-4791 | 2 | 33,3 | 15,9 | 101,9 | 7,5 | 85,9 |
| I-4790 | 1 | 29,3 | 15,5 | 103,4 | 7,1 | 87,2 |
| I-4790 | 2 | 27,9 | 15,5 | 107,8 | 7,1 | 90,9 |
| I-4792 | 1 | 33,2 | 15,4 | 102,9 | 7,0 | 86,9 |
| I-4792 | 2 | 38,0 | 15,2 | 99,8 | 6,9 | 84,3 |

| 32°C, 500 ppm urée | | | | | | |
|---|---|---|---|---|---|---|
| Ethanol Red™ | | 11,8 | 17,0 | 116,4 | 8,2 | 97,4 |
| I-4791 | 1 | 3,4 | 15,8 | 121,2 | 7,0 | 100,7 |
| I-4791 | 2 | 6,3 | 16,5 | 119,5 | 7,7 | 100,0 |
| I-4790 | 1 | 4,6 | 16,0 | 112,0 | 7,4 | 93,4 |
| I-4790 | 2 | 5,1 | 16,0 | 124,0 | 7,4 | 104,1 |
| I-4792 | 1 | 5,9 | 16,5 | 121,3 | 7,8 | 101,9 |
| I-4792 | 2 | 6,7 | 16,5 | 121,9 | 7,8 | 102,3 |

| 35°C, 500 ppm urée | | | | | | |
|---|---|---|---|---|---|---|
| Ethanol Red™ | | 21,3 | 16,0 | 116,5 | 7,5 | 98,1 |
| I-4791 | 1 | 19,8 | 15,8 | 118,5 | 7,2 | 99,6 |
| I-4791 | 2 | 19,1 | 15,9 | 119,9 | 7,3 | 101,0 |
| I-4790 | 1 | 19,1 | 15,2 | 116,3 | 6,8 | 97,8 |
| I-4790 | 2 | 19,4 | 15,0 | 115,7 | 6,6 | 97,3 |
| I-4792 | 1 | 20,3 | 15,6 | 118,7 | 7,1 | 100,2 |
| I-4792 | 2 | 20,3 | 15,8 | 118,2 | 7,3 | 99,5 |

## Revendications

1. Souche de levure *Saccharomyces cerevisiae* choisie parmi la souche de levure déposée le 25 juillet 2013 à la CNCM sous le numéro I-4791, la souche de levure déposée le 25 juillet 2013 à la CNCM sous le numéro I-4790, et la souche de levure déposée le 25 juillet 2013 sous le numéro I-4792.

2. Levure *Saccharomyces cerevisiae* obtenue par culture d'une souche de levure selon la revendication 1.

3. Utilisation d'une souche de levure selon la revendication 1 ou d'une levure selon la revendication 2 dans la production d'éthanol de première génération à partir de biomasse.

4. Utilisation selon la revendication 3, **caractérisée en ce que** la biomasse est riche en sucre, en amidon, ou en un de leurs mélanges.

5. Utilisation selon la revendication 4, **caractérisée en ce que** la biomasse comprend ou provient de la canne à sucre, de la betterave sucrière, du sorgho sucrier, du maïs, du blé, de l'orge, du seigle, du sorgho, du triticale, de la pomme de terre, de la patate douce, du manioc, ou de l'un de leurs mélanges.

6. Procédé de production d'éthanol de première génération à partir de biomasse comprenant une étape de fermentation utilisant une souche de levure selon la revendication 1 ou une levure selon la revendication 2.

7. Procédé selon la revendication 6, **caractérisé en ce que** la biomasse est riche en sucre, en amidon, ou en un de leurs mélanges.

8. Procédé selon la revendication 7, **caractérisé en ce que** la biomasse comprend de canne à sucre, de la betterave sucrière, du sorgho sucrier, du maïs, du blé, de l'orge, du seigle, du sorgho, du triticale, de la pomme de terre, de la patate douce, du manioc, ou de l'un de leurs mélanges.

## Patentansprüche

1. *Saccharomyces cerevisiae*-Hefestamm, ausgewählt aus dem Hefestamm, welcher am 25. Juli 2013 bei der CNCM unter der Nummer I-4791 hinterlegt wurde, dem Hefestamm, welcher am 25. Juli 2013 bei der CNCM unter der Nummer I-4790 hinterlegt wurde, und dem Hefestamm, welcher am 25. Juli 2013 bei der CNCM unter der Nummer I-4792 hinterlegt wurde.

2. *Saccharomyces cerevisiae*-Hefe erhalten durch Kultur eines Hefestamms nach Anspruch 1.

3. Verwendung eines Hefestamms nach Anspruch 1 oder einer Hefe nach Anspruch 2 bei der Herstellung von Ethanol der ersten Generation aus Biomasse.

4. Verwendung nach Anspruch 3, **dadurch gekennzeichnet, dass** die Biomasse reich an Zucker, an Stärke oder an einer ihrer Mischungen ist.

5. Verwendung nach Anspruch 4, **dadurch gekennzeichnet, dass** die Biomasse Zuckerrohr, Zuckerrüben, Zuckerhirse, Mais, Weizen, Gerste, Roggen, Sorghum, Triticale, Kartoffeln, Süßkartoffeln, Maniok oder eine ihrer Mischungen umfasst oder davon stammt.

6. Verfahren zur Herstellung von Ethanol der ersten Generation aus Biomasse, umfassend einen Fermentationsschritt, welcher einen Hefestamm nach Anspruch 1 oder eine Hefe nach Anspruch 2 verwendet.

7. Verfahren nach Anspruch 6, **dadurch gekennzeichnet, dass** die Biomasse reich an Zucker, an Stärke oder an einer ihrer Mischungen ist.

8. Verfahren nach Anspruch 7, **dadurch gekennzeichnet, dass** die Biomasse Zuckerrohr, Zuckerrüben, Zuckerhirse, Mais, Weizen, Gerste, Roggen, Sorghum, Triticale, Kartoffeln, Süßkartoffeln, Maniok oder eine ihrer Mischungen umfasst.

## Claims

1. *Saccharomyces cerevisiae* yeast strain chosen from the yeast strain deposited on July 25, 2013, at the CNCM under number 1-4791, the yeast strain deposited on July 25, 2013, at the CNCM under number 1-4790, and the yeast strain deposited on July 25, 2013, under number 1-4792.

2. *Saccharomyces cerevisiae* yeast obtained by culturing a yeast strain according to Claim 1.

3. Use of a yeast strain according to Claim 1 or of a yeast according to Claim 2 in the production of first-generation ethanol from biomass.

4. Use according to Claim 3, **characterized in that** the biomass is rich in sugar, in starch, or in a mixture thereof.

5. Use according to Claim 4, **characterized in that** the biomass comprises or originates from sugar cane, sugar beet, sweet sorghum, corn, wheat, barley, rye, sorghum, triticale, potato, sweet potato, cassava, or a mixture thereof.

6. Process for producing first-generation ethanol from biomass, comprising a fermentation step using a yeast strain according to Claim 1 or a yeast according to Claim 2.

7. Process according to Claim 6, **characterized in that** the biomass is rich in sugar, in starch, or in a mixture thereof.

8. Process according to Claim 7, **characterized in that** the biomass comprises sugar cane, sugar beet, sweet sorghum, corn, wheat, barley, rye, sorghum, triticale, potato, sweet potato, cassava, or a mixture thereof.
